# EUROPÄISCHE PATENTSCHRIFT

(11) **EP 1 355 614 B1**
(45) Veröffentlichungstag und Bekanntmachung des Hinweises auf die Patenterteilung: **15.03.2006**
(21) Anmeldenummer: 01971906.1
(22) Anmeldetag: 11.08.2001
(51) Int. Cl.: A61K 8/06, A61K 8/19, A61K 8/25, A61K 8/26, A61K 8/34, A61K 8/36, A61K 8/37, A61K 8/41, A61K 8/58, A61K 8/891, A61K 8/92, A61Q 19/00, A61Q 17/04, A61Q 1/02

(54) **LIPIDARME KOSMETISCHE UND DERMATOLOGISCHE ZUBEREITUNGEN IN FORM VON O/W-EMULSIONEN MIT EINEM GEHALT AN FETTSÄUREN**
LOW-LIPID COSMETIC AND DERMATOLOGICAL PREPARATIONS IN THE FORM OF O/W EMULSIONS CONTAINING FATTY ACIDS
PREPARATIONS COSMETIQUES ET DERMATOLOGIQUES PAUVRES EN LIPIDES, SOUS FORME D'EMULSIONS HUILE DANS EAU CONTENANT DES ACIDES GRAS

(30) Priorität: 07.09.2000 DE 10044313
(43) Veröffentlichungstag der Anmeldung: 29.10.2003
(73) Patentinhaber: Beiersdorf AG, 20245 Hamburg (DE)
(72) Erfinder: RIEDEL, Heidi, 22529 Hamburg (DE); LINDEMANN, Wiebke, 20257 Hamburg (DE)
(86) Internationale Anmeldenummer: PCT/EP2001/009306
(87) Internationale Veröffentlichungsnummer: WO 2002/019973

(56) Entgegenhaltungen:
- EP-A- 0 283 165
- EP-A- 0 528 380
- WO-A-00/25732
- DATABASE CHEMICAL ABSTRACTS [Online] CHEMICAL ABSTRACTS SERVICE, COLUMBUS, OHIO, US; retrieved from STN Database accession no. 133:313406 XP002200789 & JP 2000 302668 A (SAKAMOTO YAKUHIN K.K.K.) 31. Oktober 2000 (2000-10-31)

## Beschreibung

Die vorliegende Erfindung betrifft kosmetische und dermatologische Emulsionen, insbesondere hautpflegende kosmetische und dermatologische Emulsionen. In einer vorteilhaften Ausführungsform betrifft die vorliegende Erfindung eine Anwendung, welche es erlaubt, die Stabilität von fettsäurehaltigen Zubereitungen, insbesondere Emulsionen, bevorzugt von O/W-Emulsionen, zu steigern.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letzlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet Mit etwa einem Zehntel der Gesamtdicke ist sie gleichzeitig die dünnste Schicht der Haut

Die Epidermis ist ein stratifiziertes Gewebe, in dem die äußere Schicht, die Homschicht (Stratum corneum), den für die Barrierefunktion bedeutenden Teil darstellt. Das heute in der Fachwelt anerkannte Hautmodell von Elias (*P. M. Elias, Structure and Function of the Stratum Corneum Permeability Barrier, Drug Dev. Res. **13**, 1988, 97-105)* beschreibt die Homschicht als Zwei-Komponenten-System, ähnlich einer Ziegelsteinmauer (Ziegelstein-Mörtel-Modell). In diesem Modell entsprechen die Homzellen (Komeozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden.

Die vorliegende Erfindung betrifft in einer besonderen Ausführungsform kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Außer ihrer Barrierewirkung gegen externe chemische und physikalische Einflüsse tragen die epidermalen Lipide auch zum Zusammenhalt der Homschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Homschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen.

Neben der chemischen Zusammensetzung ist jedoch auch das physikalische Verhalten dieser Substanzen von Bedeutung. Daher ist die Entwicklung von sehr gut bioverträglichen Emulgatoren bzw. Tensiden wünschenswert Damit formulierte Produkte unterstützen die flüssigkristalline Organisation der Interzelluladipide des Stratum Comeums und verbessem so die Barriereeigenschaften der Homschicht. Besonders vorteilhaft ist es, wenn deren Molekülbestandteile aus natürlicherweise in der Epidermis vorkommenden Substanzen bestehen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Vertust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase) bildet Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, daß die im Überschuß eingesetzten Emulgatorgemische lamellare flüssigkristalline Phasen bzw. kristalline Gelphasen ausbilden. In der Gelnetzwerktheorie werden Stabilität und physikochemische Eigenschaften solcher Emulsionen auf die Ausbildung von viskoelastischen Gelnetzwerden zurückgeführt.

Um die Metastabilität von Emulsionen gewährleisten zu können, sind in der Regel grenzflächenaktive Substanzen, also Emulgatoren, nötig. An sich ist die Verwendung der üblichen kosmetischen Emulgatoren völlig unbedenklich. Dennoch können Emulgatoren, wie letztlich jede chemische Substanz, im Einzelfalle allergische oder auf Überempfindfichkeit des Anwenders beruhende Reaktionen hervorrufen. So ist bekannt, daß bei manchen besonders empfindlichen Personen bestimmte Lichtdermatosen durch gewisse Emulgatoren und gleichzeitige Einwirkung von Sonnenlicht ausgelöst werden.

Es ist möglich, emulgatorfreie Zubereitungen herzustellen, welche beispielsweise in einer wäßrigen Phase dispergierte Öltröpfchen, ähnlich einer O/W-Emulsion, aufweisen. Voraussetzung dafür kann sein, daß die kontinuierliche wäßrige Phase ein die dispergierte Phase stabilisierendes Gelgerüst aufweist und andere Umstände mehr. Solche Systeme werden gelegentlich Hydrodispersionen oder Oleodispersionen genannt, je nachdem, welches die disperse und welches die kontinuierliche Phase darstellt.

Es ist für die kosmetische Galenik aber weder nötig noch möglich, auf Emulgatoren ganz zu verzichten, zumal eine gewisse Auswahl an besonders milden Emulgatoren existiert Allerdings besteht ein Mangel des Standes der Technik an einer befriedigend großen Vielfalt solcher Emulgatoren, welche dann auch das Anwendungsspektrum entsprechend milder und hautverträglicher kosmetischer Zubereitungen deutlich verbreitern würde.

Physikochemisch stabile und sensorisch hochwertige Emulsionen mit niedrigen Lipidgehalt, sind schwierig zu formulieren und technisch herzustellen. Dieses gilt insbesondere für Stearat-Systeme, die als Neutralisationsmittel ausschließlich Natriumhydroxid oder aber Kaliumhydroxid enthalten.

Aufgabe war daher, all diesen den Nachteilen des Standes der Technik Abhilfe zu schaffen. Insbesondere sollten Produkte mit verringerter Klebrigkeit bzw. Schmierigkeit zur Verfügung gestellt werden. Produkte auf dem. Gebiete der pflegenden Kosmetik, der dekorativen Kosmetik und der pharmakologischen Galenik sollten gleichermaßen von den geschilderten Nachteilen des Standes der Technik befreit werden.

Weiterhin war es eine Aufgabe der Erfindung, kosmetische Grundlagen für kosmetische Zubereitungen zu entwickeln, die sich durch gute Hautverträglichkeit auszeichnen.

Bekannte kosmetische Zubereitungen sind sogenannte "Stearatemulsionen", also solchen, in welchen Stearinsäure und/oder Palmitinsäure bzw. Alkalisalze der Stearinsäure und/oder der Palmitinsäure als Emulgator wirksam sind. Diese Zubereitungen können vorteilhaft als O/W Emulsionen vorliegen und zeichnen sich durch gutes Hautgefühl aus. Nachteilig ist jedoch, daß Fettsäuren in einem pH-Bereich von 3,5 - 8,0 zur Kristallisation neigen, wodurch das angenehme Hautgefühl sowie das äußere Erscheinungsbild einer entsprechenden Zubereitung stark beeinträchtigt werden.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

Es hat sich überraschend gezeigt, und darin liegt die Lösung dieser Aufgaben, daß kosmetische oder dermatologische Zubereitungen in Form einer O/W Emulsion enthaltend
(I) 1-5 Gew.-%, bezogen auf das Gesamtgewicht der Zubereitungen, einer oder mehrerer neutralisierten bzw. teilneutralisierten C₁₄-C₃₂-Fettsäuren
(II) wobei Natriumhydroxid und/oder Kaliumhydroxid als neutralisierende Base gewählt werden, neutralisiert bzw. teilneutralisiert einem pH-Bereich der Zubereitungen von 6,0 - 8,0 entsprechend,
(III) wobei der Gehalt an alkylierten Ammoniumbasen geringer als 0,01 Gew.-% gewählt wird
(IV) 0,1 bis zu 1,5 Gew.%, bezogen auf das Gesamtgewicht der Zubereitungen, eines oder mehrerer Mono- und/oder Diester von Glycerol und/oder von Propylenglycol und/oder von Glykol,
(V) 0,5 bis zu 3 Gew.-%, bezogen auf das Gesamtgewicht der Zubereitungen, einer oder mehrere Fettalkohole, gewählt aus der Gruppe der verzweigten und unverzweigten Alkylalkohole mit 12 bis 40 Kohlenstoffatomen,
(VI) 1 bis zu 9 Gew.-%, bezogen auf das Gesamtgewicht der Zubereitungen, einer Lipidphase, zusammengesetzt aus
   (a) einem oder mehreren Lipiden mit einem Schmelzpunkt von unterhalb 30 °C
   (b) einem oder mehreren Lipiden mit einem Schmelzpunkt von oberhalb 30 °C
(VII) wobei das Verhältnis aus (a) und (b) im Bereich von 2:1 bis 6:1 liegt.
(VIII) wobei Lipide gemäß (a) 0 - 5% Silikonöl umfassen, bezogen auf das Gesamtgewicht der Zubereitungen
(IX) wobei das Verhältnis Lipide (a) : Silikonöle (VIII) vorzugsweise im Bereich von 5 :1 bis 1 : 2 liegt,

den Nachteilen des Standes der Technik abhelfen.

Es war für den Fachmann daher nicht vorauszusehen gewesen, daß die erfindungsgemäßen Zubereitungen
- besser als feuchtigkeitsspendende Zubereitungen wirken,
- besser die Hautglättung fördern,
- sich durch besser Pflegewirkung auszeichen,
- besser als Vehikel für kosmetische und medizinisch-dermatologische Wirkstoffe dienen,
- höhere Stabilität gegenüber physikalischer Zersetzung, insbesondere Phasentrennung aufweisen,
- höhere Stabilität gegenüber der Kristallisation der eingesetzten Fettsäuren aufweisen,
- sich durch bessere Bioverträglichkeit auszeichnen würden,
- sich durch besseres Hautgefühl und durch höhere kosmetische Eleganz auszeichnen würden

als die Zubereitungen des Standes der Technik. Erfindungsgemäß lassen sich sehr gute physikochemische Stabilitäten erzielen, ohne daß es nach einer längeren Lagerzeit insbesondere auch bei hohen Temperaturen zu Instabilitäten kommt (z.B. 6 Monate bei 40 °C, 2 Monate bei 50 °C, 1 Woche bei 70 °C). Hierdurch ist es möglich, Rezepturen mit überdurchschnittlich guter Hautpflege und einer herausragenden, ausgewogenen Sensorik herzustellen. Diese zeichnet sich insbesondere durch ein gutes Einzugsvermögen und durch einen auffällig geringen Rückstand aus, obwohl das Produkt während des Verteilens eine geschmeidige Reichhaltigkeit vermittelt.

Die erfindungsgemäßen Zubereitungen sind sowohl fließfähig als auch crèmeartig formulierbar, besitzen sehr gute kosmetische Eigenschaften, insbesondere was die Klebrigkeit betrifft, und weisen eine sehr gute Hautverträglichkeit sowie Hautpflegeleistung auf.

Bevorzugte Fettsäuren sind Stearinsäure und/oder Palmitinsäure (Stearin).

Vorteilhafte Ausführungsformen der vorliegenden Erfindung betreffen kosmetische und dermatologische Zubereitungen, enthaltend
(IV) 0,1 bis zu 1,5 Gew.-%, bezogen auf das Gesamtgewicht der Zubereitungen, gewählt aus der Gruppe Glycerylmonostearat, Glyceryldistearat, Propylenglycolmonostearat, Glycerylisostearat, Glyceryllanolat, Glycerylmyristat, Glyceryllaurat, Glyceryloleat, Glycerylstearatcitrat,

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt

Vorteilhaft werden der oder die Fettalkohole gewählt aus der Gruppe der einwertigen Alkohole mit 12 - 30 Kohlenstoffatomen in geraden Ketten, Dodecanol (Laurylalkohol, C₁₂H₂₅OH), Tetradecanol (Myristylalkohol, C₁₄H₂₉OH), Hexadecanol (Cetylalkohol, C₁₆H₃₃OH), Octadecanol (Stearylalkohol, C₁₈H₃₇OH), Oleylalkohol (C₁₈H₃₅OH), Eikosylalkohol (C₂₀H₃₉OH), Dokosanylalkohol (C₂₂H₄₃OH), Tetrakosanylalkohol (Camaubylalkohol, C₂₄H₄₉OH), Hexakosanylalkohol (Cerylalkohol, C₂₆H₅₃OH), Triakontylalkohol (Myricylalkohol, C₃₀H₆₁OH).

Es wird bevorzugt, die den Anteil der Siliconöle der erfindungsgemäßen Zubereitungen aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

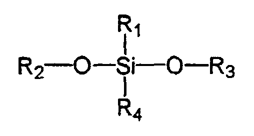

Als erfindungsgemäß vorteilhaft einzusetzenden linearen Silicone mit mehreren Siloxyleinheiten werden im allgemeinen durch Strukturelemente charakterisiert wie folgt: 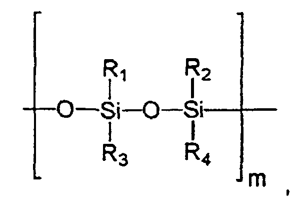
wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende cyclische Silicone werden im allgemeinen durch Strukturelemente charakterisiert, wie folgt 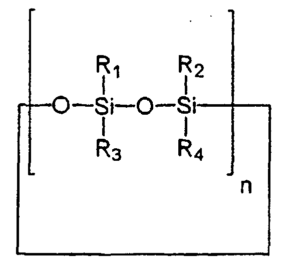
wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat

Weitere Ölkomponenten können beispielsweise gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, isopropylpalmitat, Isopropylstearat, lsopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Femer kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase der erfindungsgemäßen W/O-Emulsionen einen Gehalt an C₁₂₋₁₅-Alkylbenzoat oder aber Cetylstearylethylhexanoat aufweist oder vollständig aus diesem besteht.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhafte Ölkomponenten sind ferner beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.Die nachfolgende Tabelle 1 führt Lipide auf, die als Einzelsubstanzen oder auch im Gemisch untereinander erfindungsgemäß vorteilhaft sind. Die betreffenden Grenzflächenspannungen gegen Wasser sind in der letzten Spalte angegeben. Es ist jedoch auch vorteilhaft, Gemische aus höher- und niederpolaren und dergleichen zu verwenden.

Ferner kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Erfindungsgemäß vorteilhaft zu verwendende Lipide mit einem Schmelzpunkt von oberhalb 30 °C können vorteilhaft aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Lipide mit einem Schmelzpunkt von oberhalb 30 °C sind chemisch modifzierte Wachse und synthetische Wachse, wie beispielsweise die unter den Handelsbezeichnungen Syncrowax HRC (Glyceryltribehenat), Syncrowax HGLC (C₁₆₋₃₆ -Fettsäuretriglycerid) und Syncrowax AW 1C (C₁₈₋₃₆-Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀-Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die im Hauptanspruch geforderten Bedingungen eingehalten werden.

Erfindungsgemäß können die Lipide mit einem Schmelzpunkt von oberhalb 30 °C sowohl einzeln als auch im Gemisch vorliegen.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, lsotridecylisononanoat, Isoeicosan, 2-Ethylhexylcocoat, C₁₂₋₁₅-Alkylbenzoat, Cetylstearylethylhexanoat, Isopropylmyristat, Capryl-Caprinsäure-triglycerid, Dicaprylylether sofern die im Hauptanspruch geforderten Bedingungen eingehalten werden.

Besonders vorteilhaft sind Mischungen aus C₁₂₋₁₅-Alkybenzoat und 2-Ethylhexylisostearat, Cetearyl Ethylhexanoate, Mischungen aus Cetylstearylethylhexanoat und Isopropylmyristat sowie Mischungen aus C₁₂₋₁₅-Alkybenzoat, 2-Ethylhexylisostearat und Isotridecylisononanoat sofern die im Hauptanspruch geforderten Bedingungen eingehalten werden.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden. sofern die im Hauptanspruch geforderten Bedingungen eingehalten werden.

Als Grundbestandteile der erfindungsgemäßen Zubereitungen können verwendet werden:
- Wasser oder wäßrige Lösungen
- wäßrige ethanolische Lösungen
- natürliche Öle und/oder chemisch modifizierte natürliche Öle und/oder synthetische Öle;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäße Emulsionen im Sinne der vorliegenden Erfindung, z.B. in Form einer Hautschutzcrème, einer Hautlotion, einer kosmetischen Milch, beispielsweise in Form einer Sonnenschutzcreme oder einer Sonnenschutzmilch, sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Es ist ebenfalls von Vorteil, von den erfindungsgemäßen Eigenschaften in Form von dekorativen Kosmetika (Make-Up-Formulierungen) Gebrauch zu machen.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescremes gewöhnlich UV-A- bzw. UV-B-Filtersubstanzen eingearbeitet

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoesäure-Derivate, vorzugsweise 4-(Dimethylamino)-benzoësäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-l'-hexyloxy)-1,3,5-triazin

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bomylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Es ist vorteilhaft, den Zubereitungen im Sinne der vorliegenden Erfindung weitere antiirritative oder antientzündliche Wirkstoffe zuzugeben, insbesondere Batylalkohol (α-Octadecylglycerylether), Selachylalkohol (α-9-Octadecenylglycerylether), Chimylalkohol (α-Hexadecylglycerylether), Bisabolol und/oder Panthenol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, lmidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1-10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es kann auch gegebenenfalls vorteilhaft sein, die erfindungsgemäßen Zubereitungen als Grundlage für kosmetische oder dermatologische Zubereitungen gegen Hyperpigmentierung der Haut (z.B. als sogenannte Skin-Whitening-Produkte) zu verwenden. Es können diesen Zubereitungen die üblichen Wirkstoffe für diese Zwecke einverleibt werden, z.B. auf der Grundlage von Hydrochinon, Kojisäure, Ascorbinsäure und Azelainsäure sowie deren Derivaten.

Zubereitungen gemäß der vorliegenden Erfindung können auch Verwendung als Grundlage für kosmetische oder dermatologische Desodorantien bzw. Antitranspirantien finden. Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit Ilit, Beidellit, Nontronit, Saponit, Hectorit, Bentonit, Smectit, ferner beispielsweise Zinksalze der Ricinolsäure.

Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quatemäre Ammoniumverbindungen, Nelkenöl, Minzöl, Thymianöl, Triethylcitrat, Famesol (3,7,11-Trimethyl-2,6,10-dodecatrien-1-ol) sowie die in den Patentoffentegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Wasserphase der kosmetischen Zubereitungen im Sinne der vorliegenden Erfindung kann auch Gelcharakter aufweisen, die neben einem wirksamen Gehalt am erfindungsgemäß eingesetzten Substanzen und dafür üblicherweise verwendeten Lösungsmitteln, bevorzugt Wasser, noch weitere organische Verdickungsmittel, z.B. Gummiarabikum, Xanthangummi, Natriumalginat, Stärke und Stärkederivate (z.B. Distärkephosphat), Cellulose, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise organisch modifizierte oder auch unmodifizierte Hectorite, Bentonite, oder dergleichen, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Ferner kann es von Vorteil sein, Zubereitungen gemäß der Erfindung grenz- bzw. oberflächenaktive Agentien zuzufügen, beispielsweise kationische Emulgatoren wie insbesondere quaternäre Tenside.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, lmidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft ist auch, kationische Polymere (z.B. Jaguar® C 162 [Hydroxypropyl Guar Hydroxypropyltrimonium Chloride] bzw. modifizierten Magnesiumaluminiumsilikaten (z.B. Quaternium-18-Hectorit, welches z. B. unter der Handelsbezeichnung Bentone® 38 bei der Firma Rheox erhältlich ist, oder Stearalkonium Hectorit, welches z. B. unter der Handelsbezeichnung Softisan® Gel bei der Hüls AG erhältlich ist), einzusetzen.

Erfindungsgemäße Zubereitungen können vorteilhaft auch Ölverdickungsmittel enthalten, um die taktilen Eigenschaften der Emulsion und die Stiftkonsistenz zu verbessem. Vorteilhafte Ölverdickungsmittel im Sinne der vorliegenden Erfindung sind beispielsweise weitere Feststoffe, wie z. B. hydrophobe Siliciumoxide des Typs Aerosil®, welche von der Degussa AG erhältlich sind. Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosif® R 805, Aerosil® R 812, Aerosil® R 972, Aerosil® R 974 und/oder Aerosil® R976.

Femer sind auch sogenannte Metallseifen (d. h. die Salze höherer Fettsäuren mit Ausnahme der Alkalisalze) vorteilhafte Ölverdickungsmittel im Sinne der vorliegenden Erfindung, wie beispielsweise Aluminium-Stearat, Zink-Stearat und/oder Magnesium-Stearat

Ebenfalls vorteilhaft ist, Zubereitungen gemäß der Erfindung amphotere bzw. zwitterionische Tenside (z.B. Cocoamidopropylbetain) und Moisturizer (z.B. Betain) zuzusetzen. Vorteilhaft zu verwendende amphotere Tenside sind beispielsweise Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat, N-Alkylaminosäuren, beispielsweise Aminopropylalkylglutamid, Alkylaminopropionsäure, Natriumalkylimidodipropionat und Lauroamphocarboxyglycinat.

Die Menge der ober- bzw. grenzflächenaktiven Substanzen (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen.

### Beispiel 1 (O/W-Creme):

| | Gew.-% |
|---|---|
| Stearinsäure | 3,00 |
| Cetylalkohol | 2,50 |
| Glycerylstearat | 1,00 |
| Octyldodecanol | 3,00 |
| Cyclomethicon | 1,00 |
| Myristylmyristat | 2,00 |
| Glycerin | 5,00 |
| Carbomer | 0,10 |
| Natriumhydroxid | q.s. |
| Konservierung | q.s. |
| Parfum | q.s. |
| Wasser,demineralisiert | ad 100,00 |
| pH-Wert eingestellt auf 6,0- 8,0 | |

### Beispiel 2 (O/W-Lotion):

| | Gew.-% |
|---|---|
| Stearinsäure | 2,00 |
| Myristylalcohol | 0,50 |
| Cetylstearylalcohol | 0,50 |
| Glykolstearat | 0,25 |
| Glycerylstearat | 0,75 |
| Cetylpalmitat | 0,70 |
| Octylpalmitat | 4,20 |
| Glycerin | 3,00 |
| Carbomer | 0,10 |
| Magnesium-Aluminium-Silikat | 0,20 |
| Kaliumhydroxid | q.s. |
| Konservierung | q.s. |
| Parfum | q.s. |
| Wasser,demineralisiert | ad 100,00 |
| pH-Wert eingestellt auf 6,5-7,5 | |

### Beispiel 3 (O/W-Lotion):

| | Gew.-% |
|---|---|
| Stearinsäure | 2,00 |
| Cetylstearylalkohol | 1,50 |
| Glycerylstearat | 1,00 |
| Capryfsäure-/Caprinsäuretriglyceride | 1,00 |
| Cetylstearytethylhexanoat | 1,00 |
| Dicaprylylether | 2,00 |
| Hydriertes Cocoglycerid | 2,00 |
| Dimethicon | 2,00 |
| Xanthangummi | 0,10 |
| Magnesiumsilikat | 0,10 |
| Glycerin | 3,00 |
| Parfüm, Konservierungsmittel, | q.s. |
| Natriumhydroxid | q.s. |
| Farbstoffe usw. | q.s. |
| Wasser | ad 100,00 |
| pH-Wert eingestellt auf 7,0 - 8,0 | |

### Beispiel 4 (O/W-Emulsions-Make-up):

| | Gew.-% |
|---|---|
| Stearinsäure | 1,00 |
| Cetylalkohol | 2, 00 |
| Glykolstearat | 0,50 |
| Glycerylstearat | 0,25 |
| Cetearylisononanoat | 2,00 |
| lsopropylpalmitat | 1,00 |
| Dimethicon | 0,60 |
| Cetylpalmitat | 0,70 |
| Glycerin | 3,00 |
| Carbomer | 0,15 |
| Glimmer | 1,00 |
| Magnesiumsilikat | 1,00 |
| Eisenoxid | 1,00 |
| Titandioxid | 2,50 |
| Talkum | 5,00 |
| Vitamin A Palmitat | 0.10 |
| Natriumhydroxid | q.s. |
| Konservierung | q.s. |
| Parfum | q.s. |
| Wasser,demineralisiert | ad 100,00 |
| pH-Wert eingestellt auf 6,0 - 8,0 | |

### Beispiel 5 (O/W-Creme):

| | Gew.-% |
|---|---|
| Stearinsäure | 4,00 |
| Cetylalkohol | 2,50 |
| Glyceryldistearat | 1,00 |
| Octyldodecanol | 3,00 |
| Hydriertes Kokosfettsäureglycerid | 2,00 |
| Cyclomethicon | 2,00 |
| Vitamin E Acetat | 1,00 |
| Xanthangummi | 0,10 |
| Carbomer | 0,10 |
| Retinylpalmitat | 0,20 |
| Glycerin | 3,00 |
| BHT | 0,02 |
| Na₂H₂EDTA | 0,10 |
| Parfüm, Konservierungsmittel, | |
| Farbstoffe | q.s. |
| Kaliumhydroxid | q.s. |
| Wasser | ad 100,00 |
| pH-Wert eingestellt auf 7,0 - 8,0 | |

### Beispiel 6 (O/W-Lotion):

| | Gew.-% |
|---|---|
| Stearinsäure | 2,00 |
| Cetylstearylalkohol | 2,00 |
| Glykolstearat | 0,30 |
| Glycerylstearat | 0,30 |
| Caprylsäure-/Caprinsäuretrigtycerid | 1,00 |
| Cetylstearylethylhexanoat | 1,00 |
| Dimethicon | 4,00 |
| Myristylmyristat | 1,00 |
| Vitamin E Acetat | 2,00 |
| Natriumcarbomer | 0,10 |
| Glycerin | 3,00 |
| Parfüm, Konservierungsmittel, | |
| Farbstoffe usw. | q.s. |
| Natriumhydroxid | q.s. |
| Wasser | ad 100,00 |
| pH-Wert eingestellt auf 6,0 - 7,5 | |

### Beispiel 7 (Sonnenschutz-Creme):

| | Gew.-% |
|---|---|
| Stearinsäure | 3,50 |
| Cetylalkohol | 1,50 |
| Myristylalkohol | 1,00 |
| Glycerylstearate | 1,00 |
| Octyldodecanol | 1,00 |
| C₁₂₋₁₅ -Alkylbenzoate | 2,00 |
| Paraffinöl | 0,50 |
| Dimethicon | 1,00 |
| Cetylpalmitat | 0,80 |
| Xanthangummi | 0,10 |
| Natriumcarbomer | 0,10 |
| Glycerin | 3,00 |
| Octylmethoxycinnamat | 4,00 |
| Benzophenon-3 | 3,00 |
| Octylsalicylat | 3,00 |
| BHT | 0,02 |
| Na₂H₂EDTA | 0,10 |
| Parfüm, Konservierungsmittel, | |
| Farbstoffe, usw. | q.s. |
| Kaliumhydroxid | q.s |
| Wasser | ad 100,00 |
| pH-Wert eingestellt auf 6,5 - 8,0 | |

## Patentansprüche

1. Kosmetische oder dermatologische Zubereitungen in Form einer O/W-Emulsion enthaltend
(I) 1 - 5 Gew.-%, bezogen auf das Gesamtgewicht der Zubereitungen, einer oder mehrerer neutralisierten bzw. teilneutralisierten C₁₄-C₃₂-Fettsäuren
(II) wobei Natriumhydroxid und/oder Kaliumhydroxid als neutralisierende Base gewählt werden, neutralisiert bzw. teilneutralisiert einem pH-Bereich der Zubereitungen von 6,0 - 8,0 entsprechend,
(III) wobei der Gehalt an alkylierten Ammoniumbasen geringer als 0,01 Gew.-% gewählt wird
(IV) 0,1 bis zu 1,5 Gew.-%, bezogen auf das Gesamtgewicht der Zubereitungen, eines oder mehrerer Mono- und/oder Diester von Glycerol und/oder von Propylenglycol und/oder von Glykol,
(V) 0,5 bis zu 3 Gew.-%, bezogen auf das Gesamtgewicht der Zubereitungen, einer oder mehrere Fettalkohole, gewählt aus der Gruppe der verzweigten und unverzweigten Alkylalkohole mit 12 bis 40 Kohlenstoffatomen,
(VI) 1 bis zu 9 Gew.-%, bezogen auf das Gesamtgewicht der Zubereitungen, einer Lipidphase, zusammengesetzt aus
(a) einem oder mehreren Lipiden mit einem Schmelzpunkt von unterhalb 30 °C
(b) einem oder mehreren Lipiden mit einem Schmelzpunkt von oberhalb 30 °C
(VII) wobei das Verhältnis aus (a) und (b) im Bereich von 2:1 bis 6:1 liegt.
(VIII) wobei Lipide gemäß (a) 0-5% Silikonöl umfassen, bezogen auf das Gesamtgewicht der Zubereitungen
(IX) wobei das Verhältnis. Lipide (a) : Silikonöle (VIII) vorzugsweise im Bereich von 5:1 bis 1 : 2 liegt.

2. Zubereitungen nach Anspruch 1, enthaltend
(IV) 0,1 bis zu 1,5 Gew.-%, bezogen auf das Gesamtgewicht der Zubereitungen, gewählt aus der Gruppe Glycerylmonostearat, Glyceryldistearat, Propylenglycolmonostearat, Glycerylisostearat, Glyceryllanolat, Glycerylmyristat, Glyceryllaurat, Glyceryloleat, Glycerylstearatcitrat.

3. Zubereitungen nach Anspruch 1, enthaltend einen oder mehrere Fettalkohole gewählt aus der Gruppe der einwertigen Alkohole mit 12 - 30 Kohlenstoffatomen in geraden Ketten, Dodecanol (Laurylalkohol, C₁₂H₂₅OH), Tetradecanol (Myristylalkohol, C₁₄H₂₉OH), Hexadecanol (Cetylalkohol, C₁₆H₃₃OH), Octadecanol (Stearylalkohol, C₁₈H₃₇OH), Oleylalkohol (C₁₈H₃₅OH), Eikosylalkohol (C₂₀H₃₉OH), Dokosanylalkohol (C₂₂H₄₃OH), Tetrakosanylalkohol (Camaubylalkohol, C₂₄H₄₉OH), Hexakosanylalkohol (Cerylalkohol, C₂₆H₅₃OH), Triakontylalkohol (Myricylalkohol, C₃₀H₆₁OH).

4. Zubereitungen nach Anspruch 1, in denen die Fettsäuren Stearinsäure und/oder Palmitinsäure sind.

## Claims

1. A cosmetic or dermatological preparation in the form of a O/W emulsion comprising
(I) 1-5% by weight, based on the total weight of the preparations, of one or more neutralized or partially neutralized C₁₄-C₃₂-fatty acids
(II) where sodium hydroxide and/or potassium hydroxide are chosen as neutralizing base, corresponding to a pH range of the preparations of 6.0-8.0 in neutralized or partially neutralized form,
(III) where the content of alkylated ammonium bases is chosen to be less than 0.01% by weight
(IV) 0.1 to 1.5% by weight, based on the total weight of the preparations, of one or more mono- and/or diesters of glycerol and/or of propylene glycol and/or of glycol,
(V) 0.5 to 3% by weight, based on the total weight of the preparations, of one or more fatty alcohols chosen from the group of branched and unbranched alkyl alcohols having 12 to 40 carbon atoms,
(VI) 1 to 9% by weight, based on the total weight of the preparations, of a lipid phase composed of
(a) one or more lipids with a melting point below 30°C
(b) one or more lipids with a melting point above 30°C
(VII) where the ratio of (a) and (b) is in the range from 2:1 to 6:1.
(VIII) where lipids according to (a) comprise 0-5% of silicone oil, based on the total weight of the preparations
(IX) where the ratio of lipids (a): silicone oils (VIII) is preferably in the range from 5:1 to 1:2.

2. The preparation as claimed in claim 1, comprising
(I) 0.1 to 1.5% by weight, based on the total weight of the preparations, chosen from the group consisting of glyceryl monostearate, glyceryl distearate, propylene glycol monostearate, glyceryl isostearate, glyceryl lanolate, glyceryl myristate, glyceryl laurate, glyceryl oleate, glyceryl stearate citrate.

3. The preparation as claimed in claim 1, comprising one or more fatty alcohols chosen from the group of monohydric alcohols having 12-30 carbon atoms in straight chains, dodecanol (lauryl alcohol, C₁₂H₂₅OH), tetradecanol (myristyl alcohol, C₁₄H₂₉OH), hexadecanol (cetyl alcohol, C₁₆H₃₃OH), octadecanol (stearyl alcohol, C₁₈H₃₇OH), oleyl alcohol (C₁₈H₃₅OH), eicosyl alcohol (C₂₀H₃₉OH), docosanyl alcohol (C₂₂H₄₃OH), tetracosanyl alcohol (camaubyl alcohol, C₂₄H₄₉OH), hexacosanyl alcohol (ceryl alcohol, C₂₆H₅₃OH), triacontyl alcohol (myricyl alcohol, C₃₀H₆₁OH).

4. The preparation as claimed in claim 1 in which the fatty acids are stearic acid and/or palmitic acid.

## Revendications

1. Préparations cosmétiques ou dermatologiques sous la forme d'une émulsion H/E contenant
(I) de 1 à 5% en poids, par rapport au poids total des préparations, d'un ou plusieurs acides gras en C₁₄ à C₃₂ neutralisés ou partiellement neutralisés,
(II) de l'hydroxyde de sodium ou de l'hydroxyde de potassium étant choisi(s) comme base neutralisante, avec neutralisation ou neutralisation partielle correspondant à une plage de pH des préparations de 6,0 à 8,0,
(III) la teneur en base ammoniacale alkylée étant choisie inférieure à 0,01% en poids,
(IV) de 0,1 à 1,5% en poids, par rapport au poids total des préparations, d'un ou plusieurs mono- et/ou diesters de glycérol et/ou de propylène-glycol et/ou de glycol,
(V) de 0,5 à 3% en poids, par rapport au poids total des préparations, d'un ou plusieurs alcools gras, choisis dans le groupe formé par les alcools alkyliques ramifiés et non ramifiés comportant de 12 à 40 atomes de carbone,
(VI) de 1 à 9% en poids, par rapport au poids total des préparations, d'une phase lipidique composée de
(a) un ou plusieurs lipides d'un point de fusion inférieur à 30 °C
(b) un ou plusieurs lipides d'un point de fusion supérieur à 30 °C
(VII) la proportion de (a) et (b) étant de l'ordre de 2 : 1 à 6 : 1,
(VIII) des lipides selon (a) comprenant de 0 à 5% d'huile siliconée, par rapport au poids total des préparations,
(IX) la proportion lipides (a) : huiles siliconées (VIII) étant de préférence de l'ordre de 5 : 1 à 1 : 2.

2. Préparations selon la revendication 1, contenant
(IV) de 0,1 à 1,5% en poids, par rapport au poids total des préparations, choisi dans le groupe formé par le monostéarate de glycéryle, le distéarate de glycéryle, le monostéarate de propylèneglycol, l'isostéarate de glycéryle, le lanolate de glycéryle, le myristate de glycéryle, le laurate de glycéryle, l'oléate de glycéryle, le citrate de stéarate de glycéryle.

3. Préparations selon la revendication 1, contenant un ou plusieurs alcools gras choisis dans le groupe comprenant les alcools monovalents comportant de 12 à 30 atomes de carbone dans des chaînes linéaires, le dodécanol (alcool laurylique, C₁₂H₂₅OH), le tétradécanol (alcool myristylique, C₁₄H₂₉OH), l' hexadécanol (alcool cétylique, C₁₆H₃₃OH), l'octadécanol (alcool stéarylique, C₁₈H₃₇OH), l'alcool oléylique (C₁₈H₃₅OH), l'alcool eicosylique (C₂₀H₃₉OH), l'alcool docosanylique (C₂₂H₄₃OH), l'alcool tétracosanylique (alcool carnaubylique, C₂₄H₄₉OH), l'alcool hexacosanylique (alcool cérylique, C₂₆H₅₃OH), l'alcool triacontylique (alcool myricylique, C₃₀H₆₁OH).

4. Préparations selon la revendication 1, dans lesquelles les acides gras sont l'acide stéarique et/ou l'acide palmitique.
